# EUROPEAN PATENT SPECIFICATION

(11) **EP 4 219 455 B1**
(45) Date of publication and mention of the grant of the patent: **11.12.2024**
(21) Application number: 23166843.5
(22) Date of filing: 16.10.2020
(51) Int. Cl.: C07D 231/16

(54) **METHODS FOR THE PREPARATION OF A HALOGENATED PYRAZOLE**
VERFAHREN ZUR HERSTELLUNG EINES HALOGENIERTEN PYRAZOL
PROCÉDÉS DE PRÉPARATION D'UN PYRAZOLE HALOGÉNÉ

(30) Priority: 18.10.2019 US 201962916840 P; 27.02.2020 US 202062982248 P
(43) Date of publication of application: 02.08.2023
(62) Divisional of application: 20804069.1
(73) Proprietor: FMC Corporation, Philadelphia, Pennsylvania 19104 (US); FMC IP Technology GmbH, 8212 Neuhausen am Rheinfall (CH)
(72) Inventor: CAO, Yanchun, Philadelphia (US); LIU, Xin, Philadelphia (US); MAO, Jianhua, Philadelphia (US); XU, Zhijian, Philadelphia (US)
(74) Representative: Dehns

(56) References cited:
- WO-A1-2012/030922
- WO-A2-2008/126933
- US-A1- 2012 219 867
- US-A1- 2015 291 572
- FERNANDO BLANCO ET AL: "Substitution effects in N-pyrazole and N-imidazole derivatives along the periodic table", STRUCTURAL CHEMISTRY, KLUWER ACADEMIC PUBLISHERS-PLENUM PUBLISHERS, NE, vol. 18, no. 6, 26 September 2007 (2007-09-26), pages 965 - 975, XP019581080, ISSN: 1572-9001
- PIERCARLO FANTUCCI ET AL: "Electronic and geometrical structure of the pyrazole ligand co-ordinated to metal centres", JOURNAL OF THE CHEMICAL SOCIETY. DALTON TRANSACTIONS, no. 12, 1 January 1992 (1992-01-01), GB, pages 1981, XP055757251, ISSN: 0300-9246, DOI: 10.1039/dt9920001981
- R. MIETHCHEN R. RANDOW R. LISTEMANN J. HILDEBRANDT K. KOHLHEIM: "Micellaktivierte lodierung und partielle Dehalogenierung von Pyrarolen und 1,2,4-Triazolen Micelle Activated Reactions I. Micelle Activated Iodination and Partial Dehalogenation of Pyrazoles and 1,2,4-Triazoles", JOURNAL FÜR PRAKTISCHE CHEMIE, vol. 331, no. 5, 1 January 1989 (1989-01-01) - 1989, pages 799 - 805, XP055757253, Retrieved from the Internet <URL:https://doi.org/10.1002/prac.19893310514>

## Description

### FIELD OF INVENTION

This disclosure is directed to novel methods of synthesizing compounds of Formula II and II-A. Compounds prepared by the methods disclosed herein are useful for preparation of certain anthranilamide compounds that are of interest as insecticides, such as, for example, the insecticides chlorantraniliprole and cyantraniliprole.

### BACKGROUND

Conventional processes for the production of 5-Bromo-2-(3-chloro-pyridin-2-yl)-2H-pyrazole-3-carboxylic acid are subject to several industrial concerns, such as processability, environmental hazards, high cost, reagent reactivity, and necessary specialized equipment.

The present disclosure provides novel methods useful for preparing 5-Bromo-2-(3-chloro-pyridin-2-yl)-2H-pyrazole-3-carboxylic acid and derivatives thereof. The benefits of the methods of the present disclosure compared to previous methods are numerous and include improved overall yield, reduced cost, eliminated need for mixed solvent separations, reduced waste, simplified operation complexity, and reduced process hazards.

The disclosed methods provide an overall yield of about 50% with commercially available and easily handled reagents.

### BRIEF DESCRIPTION

In one aspect, provided herein is a method of preparing a compound of Formula II, 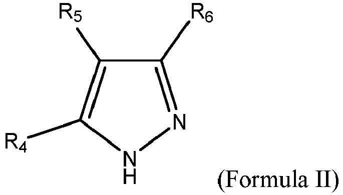 wherein each of R₄, R₅, and R₆ is independently selected from hydrogen and halogen; and wherein at least one of R₄, R₅, and R₆ is hydrogen, the method comprising
I) forming a mixture comprising
   A) a compound of Formula I, 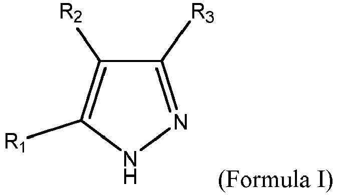
      wherein each of R₁, R₂, and R₃ is independently a halogen; and
      wherein the compound of Formula I is prepared according to a method comprising
         i) forming a mixture comprising
            a) pyrazole or a pyrazole derivative;
            b) a halogenation reagent;
            c) water;
            d) optionally a solvent; and
            e) optionally an inorganic base; and
         ii) reacting the mixture;
   B) optionally a dehalogenation reagent;
   C) a reducing agent; and
   D) a solvent; and
II) reacting the mixture.

In one aspect, provided herein is a method of preparing a compound of Formula II-A, 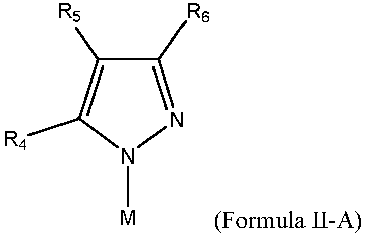
wherein M is selected from alkali metals and alkaline metals;
each of R₄, R₅, and R₆ is independently selected from hydrogen and halogen; and
wherein at least one of R₄, R₅, and R₆ is hydrogen, the method comprising
   I) forming a mixture comprising
      A) a compound of Formula I, 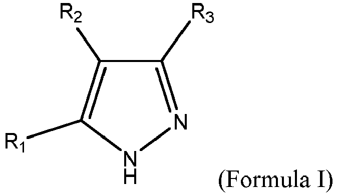
         wherein each of R₁, R₂, and R₃ is independently a halogen; and
         wherein the compound of Formula I is prepared according to a method comprising
            i) forming a mixture comprising
               a) pyrazole or a pyrazole derivative;
               b) a halogenation reagent;
               c) water;
               d) optionally a solvent; and
               e) optionally an inorganic base; and
            ii) reacting the mixture;
      B) optionally a dehalogenation reagent;
      C) a reducing agent; and
      D) a solvent; and
   II) reacting the mixture.

A compound of Formula II-A is prepared in a method of the invention: 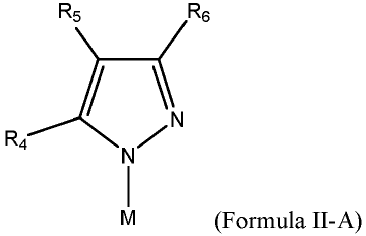
wherein M is selected from alkali metals and alkaline metals;
each of R₄, R₅, and R₆ is independently selected from hydrogen and halogen; and
wherein at least one of R₄, R₅, and R₆ is hydrogen.

### DETAILED DESCRIPTION OF THE DISCLOSURE

As used herein, the terms "comprises," "comprising," "includes," "including," "has," "having," "contains", "containing," "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process or method.

The transitional phrase "consisting of" excludes any element, step, or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of" is used to define a composition or method that includes materials, steps, features, components, or elements, in addition to those literally disclosed, provided that these additional materials, steps, features, components, or elements do not materially affect the basic and novel characteristic(s) of the claimed invention. The term "consisting essentially of" occupies a middle ground between "comprising" and "consisting of".

Where an invention or a portion thereof is defined with an openended term such as "comprising," it should be readily understood that (unless otherwise stated) the description should be interpreted to also describe such an invention using the terms "consisting essentially of" or "consisting of."

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

As used herein, the term "about" means plus or minus 10% of the value.

The term "halogen", either alone or in compound words such as "haloalkyl", includes fluorine, chlorine, bromine or iodine. Further, when used in compound words such as "haloalkyl", said alkyl may be partially or fully substituted with halogen atoms which may be the same or different.

When a group contains a substituent which can be hydrogen, for example R⁴, then, when this substituent is taken as hydrogen, it is recognized that this is equivalent to said group being unsubstituted.

The term "organic base" includes, without limitation, amine compounds (e.g., primary, secondary and tertiary amines), heterocycles including nitrogen-containing heterocycles, and ammonium hydroxide.

The term "inorganic base" includes, without limitation, inorganic compounds with the ability to react with, or neutralize, acids to form salts, such as, for example, metal salts of hydroxide, carbonate, bicarbonate and phosphate.

The term "halogenation reagent" includes, without limitation, halogens and inorganic compounds, such as, for example, bromine, NBS, and 1,3-dibromo-5,5-dimethylhylhydantoin.

The term "phase transfer catalyst" includes compounds that facilitate the migration of a reactant from one phase into another phase where a reaction occurs. Phase transfer catalysis refers to the acceleration of the reaction upon the addition of the phase transfer catalyst.

The term "ester" includes, without limitation, a functional group comprising an ester bond (C(=O)-O-). In some aspects, the functional group comprising an ester bond is an alkyl (or cycloalkyl) having one to eight carbon atoms, like methyl, ethyl, 1 -propyl, 2-propyl, 1 - butyl, 1-methylheptyl (meptyl), etc.

The term "ether" includes, without limitation, a functional group comprising an ether bond (C-O-C).

The term "nitrile" includes, without limitation, a functional group comprising a nitrile bond (-C≡N).

The term "carboxylic acid" includes, without limitation, a functional group comprising a carboxylic acid bond (C(=O)-OH).

The term "organic acid" includes, without limitation, a functional group that confers acidity and consists of atoms selected from carbon, nitrogen, oxygen, and hydrogen.

Certain compounds formed in this invention can exist as one or more stereoisomers. The various stereoisomers include enantiomers, diastereomers, atropisomers and geometric isomers. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers.

Embodiments of this invention include Embodiments 60-67 and Embodiments 110-119 and the other embodiments disclosed below are for reference only.

Compounds of Formula II prepared according to the methods of the invention can be used to prepare compounds of Formula III according to a method as defined in any of Embodiments 17-22 below. Compounds of Formula III can be used to prepare compounds of Formula VI according to a method as defined in any of Embodiments 1-16 below.

Embodiment 1. A method of preparing a compound of Formula VI, wherein 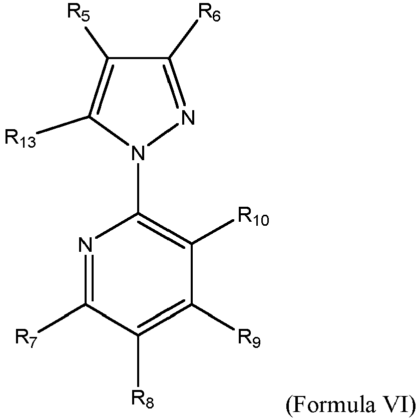
each of R₅ - R₁₀ is independently selected from hydrogen and halogen; and
R₁₃ is an organic acid, the method comprising
   I) forming a mixture comprising
      A) a compound of Formula V, wherein 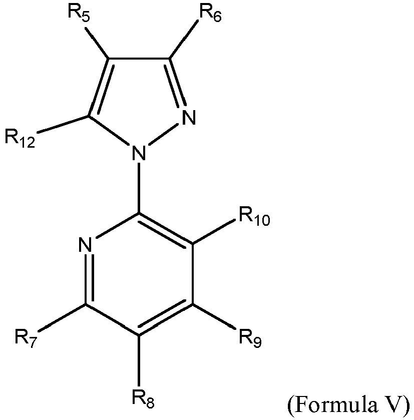
         each of R₅ - R₁₀ is independently selected from hydrogen and halogen;
         R₁₂ is selected from ether, ester, and nitrile; and
         wherein the compound of Formula V is prepared according to a method comprising
            i) forming a mixture comprising
               a) a compound of Formula III, wherein 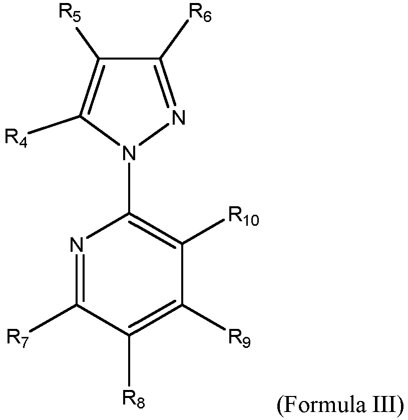 each of R₄ - R₁₀ is independently selected from hydrogen and halogen; and wherein at least one of R₄, R₅, and R₆ is hydrogen;
               b) a first solvent;
               c) a second solvent;
               d) a compound comprising a metal; and
               e) optionally an additive; and
            ii) reacting the mixture; and
      B) a metal hydroxide; and
   II) reacting the mixture.

Embodiment 2. The method of embodiment 1, wherein the metal hydroxide is selected from alkali hydroxide, alkaline earth metal hydroxide, and combinations thereof.

Embodiment 3. The method of embodiment 2, wherein the alkali hydroxide is selected from lithium hydroxide, sodium hydroxide, and potassium hydroxide.

Embodiment 4. The method of embodiment 2, wherein the alkaline earth metal hydroxide is selected from calcium hydroxide and barium hydroxide.

Embodiment 5. The method of embodiment 1, wherein the method step II) of reacting the mixture occurs at a reaction temperature in the range of about 0 °C to about 90 °C.

Embodiment 6. The method of embodiment 1, wherein the compound comprising a metal is selected from a Grignard reagent, and a lithium-containing compound.

Embodiment 7. The method of embodiment 6, wherein the Grignard reagent is selected from MeMgCl, iPrMgCl, iPrMgBr, EtMgCl, and combinations thereof.

Embodiment 8. The method of embodiment 7, wherein the Grignard reagent is iPrMgBr.

Embodiment 9. The method of embodiment 6, wherein the lithium-containing compound is nBuLi.

Embodiment 10. The method of embodiment 1, wherein the first solvent is selected from THF, toluene, 1,4-dioxane, Me-THF, and combinations thereof.

Embodiment 11. The method of embodiment 10, wherein the first solvent is THF.

Embodiment 12. The method of embodiment 1, wherein the second solvent is selected from dimethyl carbonate, N,N-dimethyacetamide, and combinations thereof.

Embodiment 13. The method of embodiment 12, wherein the second solvent is dimethyl carbonate.

Embodiment 14. The method of embodiment 1, wherein the method step ii) of reacting the mixture occurs at a reaction temperature in the range of about 0 °C to about 60 °C.

Embodiment 15. The method of embodiment 14, wherein the method step ii) of reacting the mixture occurs at a reaction temperature in the range of about 0 °C to about 30 °C.

Embodiment 16. The method of embodiment 1, wherein R₄, R₅, and R₆ of Formula III are each independently hydrogen.

Embodiment 17. The method of embodiment 1, wherein the compound of Formula III is prepared according to a method comprising
I) forming a mixture comprising
   A) a compound of Formula II, wherein 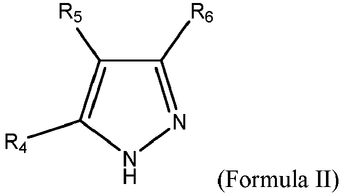
      each of R₄, R₅, and R₆ is independently selected from hydrogen and halogen; and
      wherein at least one of R₄, R₅, and R₆ is hydrogen;
   B) a compound of Formula IV, wherein 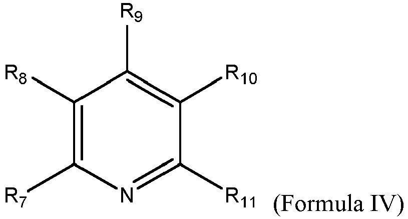 each of R₇ - R₁₁ is independently selected from hydrogen and halogen;
   C) a solvent;
   D) an inorganic base; and
   E) optionally an additive; and
II) reacting the mixture.

Embodiment 18. The method of embodiment 17, wherein the inorganic base is selected from powder sodium hydroxide, powder potassium hydroxide, potassium carbonate, sodium carbonate, potassium phosphate, powder sodium methoxide, powder potassium t-butoxide, and combinations thereof.

Embodiment 19. The method of embodiment 17, wherein the solvent is selected from toluene, N,N-dimethylformamide, N,N-dimethylacetamide, N-methyl-2-pyrrolidone, NMP, sulfolane, triglyme, diglyme and combinations thereof.

Embodiment 20. The method of embodiment 17, wherein the additive is selected from potassium iodide, a phase transfer catalyst, and combinations thereof.

Embodiment 21. The method of embodiment 17, wherein the phase transfer catalyst is selected from butyl ammonium chloride, tetra butyl ammonium bromide, aliquat-336, 18-crown-6, and combinations thereof.

Embodiment 22. The method of embodiment 17, wherein the method step II) of reacting the mixture occurs at a reaction temperature in the range of about 140 °C to about 200 °C.

Compounds of Formula III as disclosed herein can be used to prepare compounds of Formula V according to a method as defined in any of Embodiments 32-43 below.

Embodiment 32. A method of preparing a compound of Formula V, wherein 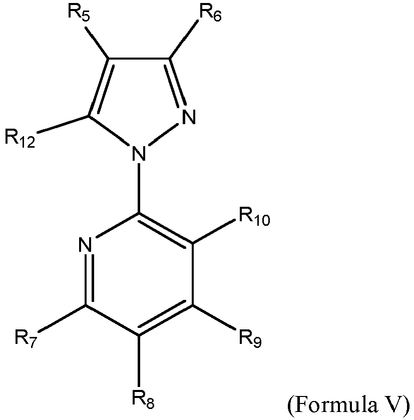
each of R₅ - R₁₀ is independently selected from hydrogen and halogen; and
R₁₂ is selected from ether, ester, and nitrile, the method comprising
   I) forming a mixture comprising
      A) a compound of Formula III, wherein 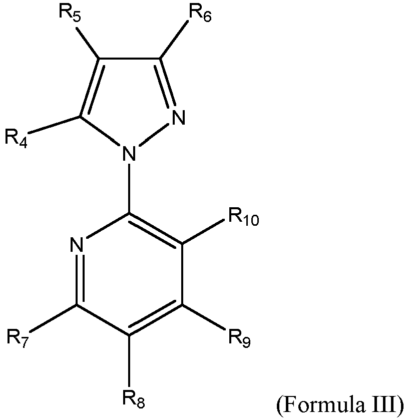
         R₄ is a halogen; and
         each of R₅ - R₁₀ is independently selected from hydrogen and halogen;
      B) a first solvent;
      C) a second solvent;
      D) a compound comprising a metal; and
      E) optionally an additive; and
   II) reacting the mixture.

Embodiment 33. The method of embodiment 32, wherein the compound comprising a metal is selected from a Grignard reagent and a lithium-containing compound.

Embodiment 34. The method of embodiment 33, wherein the Grignard reagent is selected from MeMgCl, iPrMgCl, iPrMgBr, EtMgCl, and combinations thereof.

Embodiment 35. The method of embodiment 34, wherein the Grignard reagent is iPrMgBr.

Embodiment 36. The method of embodiment 33, wherein the lithium-containing compound is nBuLi.

Embodiment 37. The method of embodiment 32, wherein the first solvent is selected from THF, toluene, 1,4-dioxane, Me-THF, and combinations thereof.

Embodiment 38. The method of embodiment 37, wherein the first solvent is THF.

Embodiment 39. The method of embodiment 32, wherein the second solvent is selected from dimethyl carbonate, N,N-dimethyacetamide, and combinations thereof.

Embodiment 40. The method of embodiment 39, wherein the second solvent is dimethyl carbonate.

Embodiment 41. The method of embodiment 32, wherein the method step II) of reacting the mixture occurs at a reaction temperature in the range of about 0 °C to about 60 °C.

Embodiment 42. The method of embodiment 41, wherein the method step II) of reacting the mixture occurs at a reaction temperature in the range of about 0 °C to about 30 °C.

Embodiment 43. The method of embodiment 32, wherein R₄, R₅, and R₆ of Formula III are each independently hydrogen.

Embodiment 60. A method of preparing a compound of Formula II, 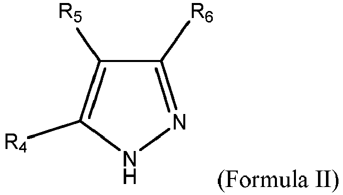
wherein each of R₄, R₅, and R₆ is independently selected from hydrogen and halogen; and
wherein at least one of R₄, R₅, and R₆ is hydrogen, the method comprising
   I) forming a mixture comprising
      A) a compound of Formula I, 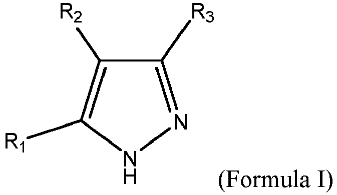
         wherein each of R₁, R₂, and R₃ is independently a halogen; and
         wherein the compound of Formula I is prepared according to a method comprising
            i) forming a mixture comprising
               a) pyrazole or a pyrazole derivative;
               b) a halogenation reagent;
               c) water;
               d) optionally a solvent; and
               e) optionally an inorganic base; and
            ii) reacting the mixture;
      B) optionally a dehalogenation reagent;
      C) a reducing agent; and
      D) a solvent; and
   II) reacting the mixture.

Embodiment 61-1. The method of embodiment 60, wherein the solvent in d) is selected from alkanes, ethers, halogenated solvents, esters, water, and combinations thereof. In some embodiments, the halogenated solvent is selected from dichloromethane, methylene chloride, trichloroethylene, and combinations thereof. In some embodiments, the ester solvent is selected from butyl acetate, sec-butyl acetate, tert-butyl acetate, and combinations thereof. In some embodiments, the solvent is an ether solvent selected from methyl tert-butyl ether (MTBE), diethyl ether (Et2O), and combinations thereof.

Embodiment 61-11. The method of embodiment 60, wherein the solvent D) is selected from acetic acid, water, toluene, N,N-dimethylformamide, N,N-dimethylacetamide, and combinations thereof.

Embodiment 62. The method of embodiment 60, wherein the reducing agent is selected from sodium sulfite, sodium bisulfite, sodium hyposulfite, sodium thiosulfate, sodium hydrosulfide, sodium sulfate, and combinations thereof.

Embodiment 63. The method of embodiment 60, wherein the dehalogenation reagent is selected from sodium iodide, iodine, potassium iodide, tetra-n-butyl ammonium iodide, and combinations thereof.

Embodiment 64. The method of embodiment 60, wherein the method step II) of reacting the mixture occurs at a reaction temperature in the range of about 100 °C to about 180 °C.

Embodiment 65. The method of embodiment 60, wherein the halogenation reagent comprises
A) a reagent selected from hydrogen bromide, bromine, N-bromosuccinimide, 1,3-dibromo-5,5-dimethylhylhydantoin, sodium bromide, potassium bromide, and combinations thereof; and
B) optionally hydrogen peroxide.

Embodiment 66. The method of embodiment 60, wherein the inorganic base is selected from powder sodium hydroxide, sodium hydroxide solution, powder sodium acetate, and combinations thereof.

Embodiment 67. The method of embodiment 60, wherein the method step ii) of reacting the mixture occurs at a reaction temperature in the range of about 0 °C to about 70 °C.

Compounds of Formula II prepared according to the methods of the invention can be used to prepare compounds of Formula III according to a method as defined in any of Embodiments 82-87 below. Compounds of Formula III can be used to prepare compounds of Formula VI according to a method as defined in any of Embodiments 68-81 below.

Embodiment 68. A method of preparing a compound of Formula VI, wherein 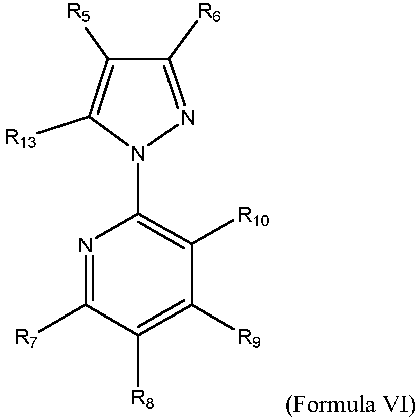
each of R₅ - R₁₀ is independently selected from hydrogen and halogen; and
R₁₃ is an organic acid, the method comprising
   I) forming a mixture comprising
      A) a compound of Formula V, wherein 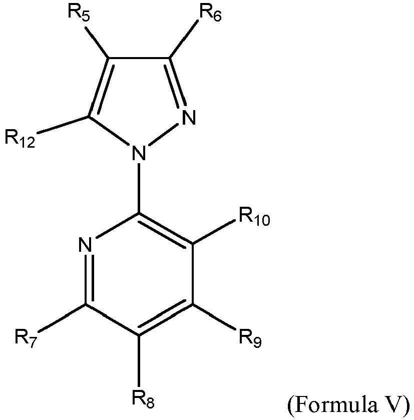
         each of R₅ - R₁₀ is independently selected from hydrogen and halogen;
         R₁₂ is selected from ether, ester, and nitrile; and
         wherein the compound of Formula V is prepared according to a method comprising
            i) forming a mixture comprising
               a) a compound of Formula III, wherein 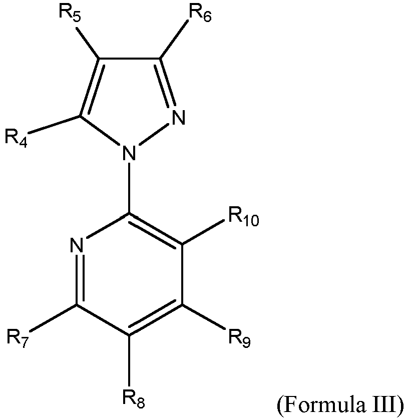 each of R₄ - R₁₀ is independently selected from hydrogen and halogen; and wherein at least one of R₄, R₅, and R₆ is hydrogen;
               b) a cyanide reagent;
               c) a solvent;
               d) a compound comprising a metal; and
               e) optionally an additive; and
            ii) reacting the mixture; and
      B) an acid; and
   II) reacting the mixture.

Embodiment 69. The method of embodiment 68, wherein the acid is selected from H₂SO₄, HCl, and combinations thereof.

Embodiment 70. The method of embodiment 68, wherein the method step II) of reacting the mixture occurs at a reaction temperature in the range of about 50 °C to about 100 °C.

Embodiment 71. The method of embodiment 68, wherein the compound comprising a metal is a transition metal catalyst.

Embodiment 72. The method of embodiment 71, wherein the transition metal catalyst is cuprous iodide.

Embodiment 73. The method of embodiment 68, wherein the cyanide reagent is selected from sodium cyanide, copper(I) cyanide, zinc cyanide, and combinations thereof.

Embodiment 74. The method of embodiment 73, wherein the cyanide reagent is sodium cyanide.

Embodiment 75. The method of embodiment 68, wherein the solvent is selected from N-methyl-2-pyrrolidone (NMP), acetonitrile, diglyme, triglyme, ethylene glycol, propylene glycol, ethanol, isobutanol, and alcohols and sulfolane, dimethyl carbonate, N,N-dimethyacetamide, and combinations thereof.

Embodiment 76. The method of embodiment 75, wherein the solvent is N,N-dimethyacetamide or diglyme.

Embodiment 77. The method of embodiment 68, wherein the method step ii) of reacting the mixture occurs at a reaction temperature in the range of about 120 °C to about 150 °C.

Embodiment 78. The method of embodiment 77, wherein the method step ii) of reacting the mixture occurs at a reaction temperature in the range of about 130 °C to about 140 °C.

Embodiment 79. The method of embodiment 68, wherein the additive is selected from potassium iodide, a phase transfer catalyst, and combinations thereof.

Embodiment 80. The method of embodiment 79, wherein the additive is potassium iodide.

Embodiment 81. The method of embodiment 68, wherein R₄, R₅, and R₆ of Formula III are each independently hydrogen.

Embodiment 82. The method of embodiment 68, wherein the compound of Formula III is prepared according to a method comprising
I) forming a mixture comprising
   A) a compound of Formula II, wherein 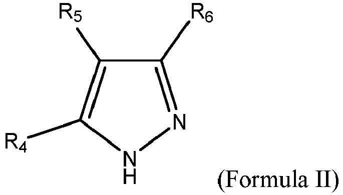
      each of R₄, R₅, and R₆ is independently selected from hydrogen and halogen; and
      wherein at least one of R₄, R₅, and R₆ is hydrogen;
   B) a compound of Formula IV, wherein 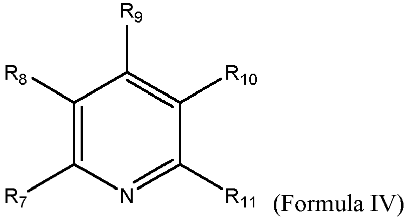 each of R₇ - R₁₁ is independently selected from hydrogen and halogen;
   C) a solvent;
   D) an inorganic base; and
   E) optionally an additive; and
II) reacting the mixture.

Embodiment 83. The method of embodiment 82, wherein the inorganic base is selected from powder sodium hydroxide, powder potassium hydroxide, potassium carbonate, potassium phosphate, powder sodium methoxide, powder potassium t-butoxide, and combinations thereof.

Embodiment 84. The method of embodiment 82, wherein the solvent is selected from toluene, N,N-dimethylformamide, N,N-dimethylacetamide, N-methyl-2-pyrrolidone, and combinations thereof.

Embodiment 85. The method of embodiment 82, wherein the additive is selected from potassium iodide, a phase transfer catalyst, and combinations thereof.

Embodiment 86. The method of embodiment 85, wherein the phase transfer catalyst is selected from butyl ammonium chloride, tetra butyl ammonium bromide, aliquat-336, 18-crown-6, and combinations thereof.

Embodiment 87. The method of embodiment 82, wherein the method step II) of reacting the mixture occurs at a reaction temperature in the range of about 140 °C to about 200 °C.

Compounds of Formula III as disclosed herein can be used to prepare compounds of Formula V according to a method as defined in any of Embodiments 97-109 below.

Embodiment 97. A method of preparing a compound of Formula V, wherein 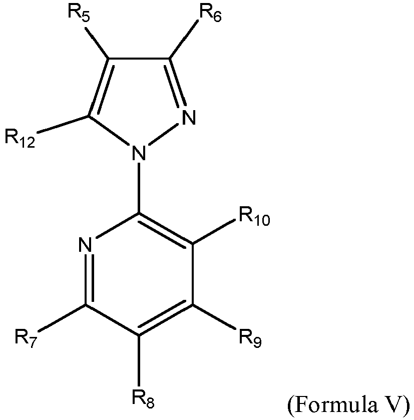
each of R₅ - R₁₀ is independently selected from hydrogen and halogen; and
R₁₂ is selected from ether, ester, and nitrile, the method comprising
   I) forming a mixture comprising
      A) a compound of Formula III, wherein 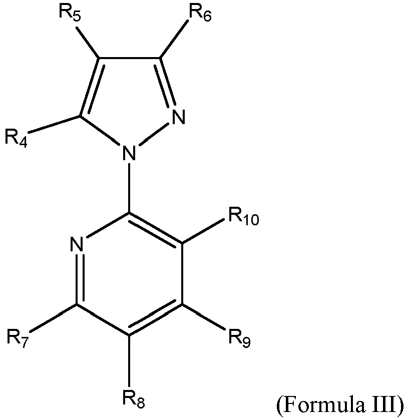
         R₄ is a halogen; and
         each of R₅ - R₁₀ is independently selected from hydrogen and halogen;
      B) a cyanide reagent;
      C) a solvent;
      D) a compound comprising a metal; and
      E) optionally an additive; and
   II) reacting the mixture.

Embodiment 98. The method of embodiment 97, wherein the compound comprising a metal is a transition metal catalyst.

Embodiment 99. The method of embodiment 98, wherein the transition metal catalyst is selected from cuprous iodide, cuprous bromide, cuprous oxide, and combinations thereof.

Embodiment 100. The method of embodiment 99, wherein the transition metal catalyst is cuprous iodide.

Embodiment 101. The method of embodiment 97, wherein the cyanide reagent is selected from sodium cyanide, copper(I) cyanide, zinc cyanide, and combinations thereof.

Embodiment 102. The method of embodiment 101, wherein the cyanide reagent is sodium cyanide.

Embodiment 103. The method of embodiment 97, wherein the solvent is selected from N-methyl-2-pyrrolidone (NMP), acetonitrile, diglyme, triglyme, ethylene glycol, propylene glycol, ethanol, isobutanol, and alcohols and sulfolane, dimethyl carbonate, N,N-dimethyacetamide, and combinations thereof.

Embodiment 104. The method of embodiment 103, wherein the solvent is N,N-dimethyacetamide or diglyme.

Embodiment 105. The method of embodiment 97, wherein the method step II) of reacting the mixture occurs at a reaction temperature in the range of about 120 °C to about 150 °C.

Embodiment 106. The method of embodiment 105, wherein the method step II) of reacting the mixture occurs at a reaction temperature in the range of about 130 °C to about 140 °C.

Embodiment 107. The method of embodiment 97, wherein the additive is selected from potassium iodide, a phase transfer catalyst, and combinations thereof.

Embodiment 108. The method of embodiment 107, wherein the additive is potassium iodide.

Embodiment 109. The method of embodiment 97, wherein R₄, R₅, and R₆ of Formula III are each independently hydrogen.

Embodiment 110. A method of preparing a compound of Formula II-A, 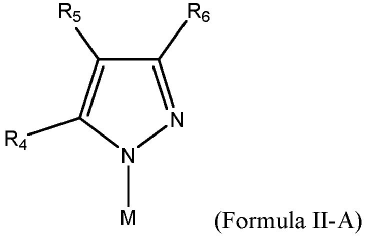
wherein M is selected from alkali metals and alkaline metals;
each of R₄, R₅, and R₆ is independently selected from hydrogen and halogen; and
wherein at least one of R₄, R₅, and R₆ is hydrogen, the method comprising
   I) forming a mixture comprising
      A) a compound of Formula I, 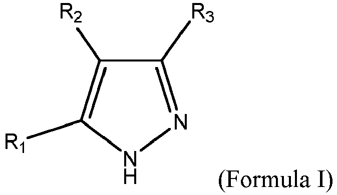
         wherein each of R₁, R₂, and R₃ is independently a halogen; and
         wherein the compound of Formula I is prepared according to a method comprising
            i) forming a mixture comprising
               a) pyrazole or a pyrazole derivative;
               b) a halogenation reagent;
               c) water; and
               d) optionally an inorganic base; and
            ii) reacting the mixture;
      B) optionally a dehalogenation reagent;
      C) a reducing agent; and
      D) a solvent; and
   II) reacting the mixture.

Embodiment 111-I. The method of embodiment 110, wherein the solvent in d) is selected from alkanes, ethers, halogenated solvents, esters, water, and combinations thereof. In some embodiments, the halogenated solvent is selected from dichloromethane, methylene chloride, trichloroethylene, and combinations thereof. In some embodiments, the ester solvent is selected from butyl acetate, sec-butyl acetate, tert-butyl acetate, and combinations thereof. In some embodiments, the solvent is an ether solvent selected from methyl tert-butyl ether (MTBE), diethyl ether (Et2O), and combinations thereof.

Embodiment 111 -II. The method of embodiment 110, wherein the solvent D) is selected from acetic acid, water, toluene, N,N-dimethylformamide, N,N-dimethylacetamide, and combinations thereof.

Embodiment 112. The method of embodiment 110, wherein the reducing agent is selected from sodium sulfite, sodium bisulfite, sodium hyposulfite, sodium thiosulfate, sodium hydrosulfide, sodium sulfate, and combinations thereof.

Embodiment 113. The method of embodiment 110, wherein the dehalogenation reagent is selected from sodium iodide, iodine, potassium iodide, tetra-n-butyl ammonium iodide, and combinations thereof.

Embodiment 114. The method of embodiment 110, wherein the method step II) of reacting the mixture occurs at a reaction temperature in the range of about 100 °C to about 180 °C.

Embodiment 115. The method of embodiment 110, wherein the halogenation reagent comprises
A) a reagent selected from hydrogen bromide, bromine, N-bromosuccinimide, 1,3-dibromo-5,5-dimethylhylhydantoin, sodium bromide, potassium bromide, and combinations thereof; and
B) optionally hydrogen peroxide.

Embodiment 116. The method of embodiment 110, wherein the inorganic base is selected from powder sodium hydroxide, sodium hydroxide solution, powder sodium acetate, and combinations thereof.

Embodiment 117. The method of embodiment 110, wherein the method step ii) of reacting the mixture occurs at a reaction temperature in the range of about 0 °C to about 70 °C.

Embodiment 118. The method of embodiment 110, wherein M is selected from lithium, sodium, potassium, calcium, and magnesium.

Embodiment 119. The method of embodiment 110, wherein the compound of Formula II-A is

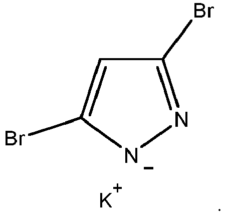

A compound of Formula VI may be prepared according to a method represented by Scheme 1. The R groups are as defined anywhere in this disclosure.

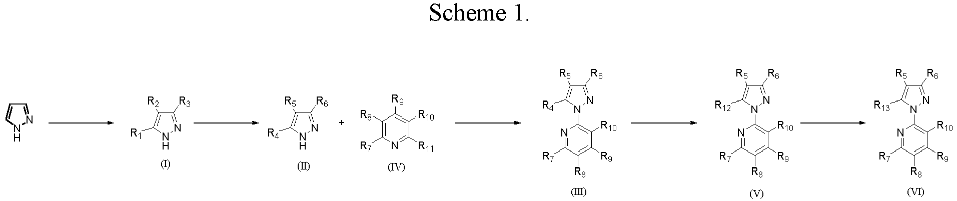

5-Bromo-2-(3-chloro-pyridin-2-yl)-2H-pyrazole-3-carboxylic acid may be prepared according to a method represented by Scheme 2.

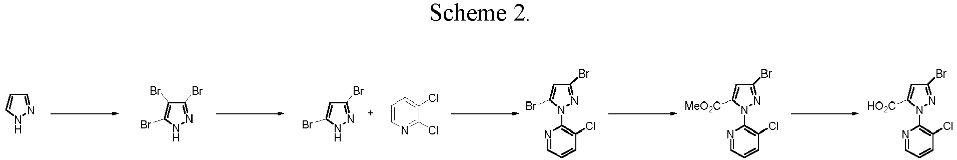

5-Bromo-2-(3-chloro-pyridin-2-yl)-2H-pyrazole-3-carboxylic acid may be prepared according to a method represented by Scheme 3.

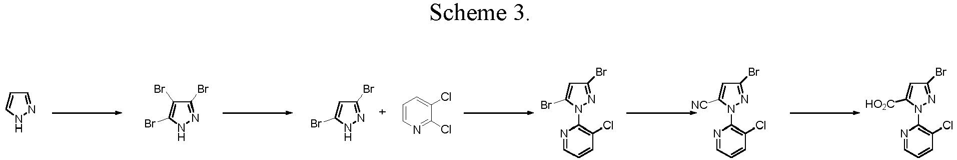

In one aspect, a compound of Formula I is prepared according to a method represented by Scheme 4. The R groups are as defined anywhere in this disclosure.

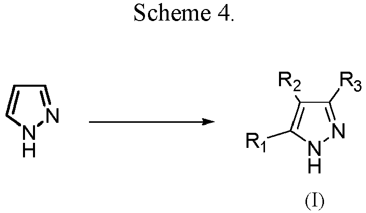

This aspect includes reacting pyrazole with a halogenation reagent in water, and optionally a solvent and further optionally in the presence of an inorganic base. In one embodiment, the halogenation reagent is selected from hydrogen peroxide/HBr, Bromine (Br₂), N-bromosuccinimide, 1,3-dibromo-5,5-dimethylhylhydantoin, hydrogen peroxide/NaBr, hydrogen peroxide/KBr, hydrogen peroxide/Br₂, and combinations thereof. In another embodiment, the halogenation reagent is hydrogen peroxide/HBr. In one embodiment, inorganic base is selected from powder sodium hydroxide, sodium hydroxide solution, powder sodium acetate, and combinations thereof. In another embodiment there is no base. In one embodiment, the reaction temperature is in the range from about 0 °C to about 70 °C. In another embodiment, the reaction temperature is in the range from about 0 °C to about 30 °C. In yet another embodiment, the solvent is selected from alkanes, ethers, halogenated solvents, esters, water, and combinations thereof. In some embodiments, the solvent is an ether solvent selected from methyl tert-butyl ether (MTBE), diethyl ether (Et2O), and combinations thereof.

In one aspect, a compound of Formula II is prepared according to a method represented by Scheme 5. The R groups are as defined anywhere in this disclosure.

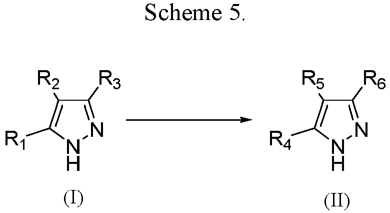

This aspect includes reacting a compound of Formula I with a dehalogenation reagent in a solvent in the presence of a reducing agent. In one embodiment, the solvent is selected form aromatic solvents, halogenated aromatic solvents, and combinations thereof. In one embodiment, the solvent is selected from acetic acid, water, toluene, N,N-dimethylformamide (DMF), N,N-dimethylacetamide (DMAc), N-methyl-2-pyrrolidone (NMP), sulfolane and combinations thereof. In another embodiment, the solvent is N,N-dimethylacetamide (DMAc). In yet another embodiment, the solvent is sulfolane. In one embodiment, the dehalogenation reagent is selected from sodium iodide, iodine, potassium iodide, tetra-n-butyl ammonium iodide (TBAI), and combinations thereof. In another embodiment, the dehalogenation reagent is potassium iodide. In one embodiment, the reducing agent is selected from sodium sulfite, sodium bisulfite, sodium hyposulfite, sodium thiosulfate, sodium hydrosulfide, sodium sulphate, and combinations thereof. In another embodiment, the reducing agent is sodium sulfite. In one embodiment, the reaction temperature is in the range from about 100 °C to about 180 °C. In another embodiment, the reaction temperature is in the range from about 160 °C to about 180 °C.

In one aspect, a compound of Formula II-A is prepared according to a method represented by Scheme 6. The R groups are as defined anywhere in this disclosure.

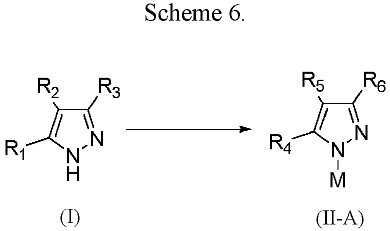

The compound of Formula II-A is a metal salt of Formula II. In one embodiment, the bond between M and N is an ionic bond. M is selected from alkali metals and alkaline metals. In one embodiment, M is selected from lithium, sodium, and potassium. In another embodiment, M is potassium. In another embodiment, M is selected from calcium and magnesium.

In one embodiment, the compound of Formula II-A is

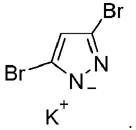

This aspect includes reacting a compound of Formula I with a dehalogenation reagent in a solvent in the presence of a reducing agent. In one embodiment, the solvent is selected form aromatic solvents, halogenated aromatic solvents, and combinations thereof. In one embodiment, the solvent is selected from acetic acid, water, toluene, N,N-dimethylformamide (DMF), N,N-dimethylacetamide (DMAc), N-methyl-2-pyrrolidone (NMP), sulfolane and combinations thereof. In another embodiment, the solvent is N,N-dimethylacetamide (DMAc). In yet another embodiment, the solvent is sulfolane. In one embodiment, the dehalogenation reagent is selected from sodium iodide, iodine, potassium iodide, tetra-n-butyl ammonium iodide (TBAI), and combinations thereof. In another embodiment, the dehalogenation reagent is potassium iodide. In one embodiment, the reducing agent is selected from sodium sulfite, sodium bisulfite, sodium hyposulfite, sodium thiosulfate, sodium hydrosulfide, sodium sulphate, and combinations thereof. In another embodiment, the reducing agent is sodium sulfite. In one embodiment, the reaction temperature is in the range from about 100 °C to about 180 °C. In another embodiment, the reaction temperature is in the range from about 160 °C to about 180 °C.

A compound of Formula III may be prepared according to a method represented by Scheme 7. The R groups are as defined anywhere in this disclosure.

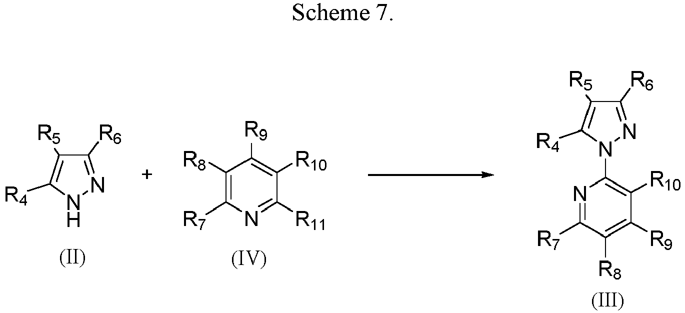

This method includes the step of mixing a compound of Formula II with a compound of Formula IV in a solvent in the presence of an inorganic base and optionally an additive. The inorganic base may be selected from powder sodium hydroxide, powder potassium hydroxide, sodium carbonate, potassium carbonate, potassium phosphate powder sodium methoxide, powder potassium *t*-butoxide, and combinations thereof, such as sodium carbonate. The solvent may be selected from toluene, N,N-dimethylformamide (DMF), N,N-dimethylacetamide (DMAc), N-methyl-2-pyrrolidone (NMP), sulfolane, diglyme, triglyme and combinations thereof, such as N,N-dimethyl-acetamide (DMAc) or sulfolane. The additive may be a phase catalyst selected from butyl ammonium chloride (TBAC), tetra butyl ammonium bromide (TBAB), aliquat-336, 18-crown-6, and combinations thereof, such as 18-crown-6. The additive may be potassium iodide. The reaction temperature may be in the range from about 140 °C to about 200 °C. The temperature may be in the range from about 155 °C to about 175 °C.

A compound of Formula V may be prepared according to a method represented by Scheme 8. The R groups are as defined anywhere in this disclosure.

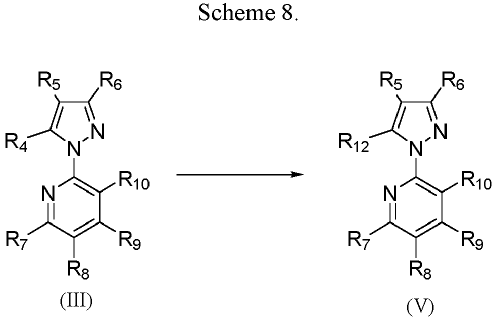

This method includes mixing a compound of Formula III with dimethyl carbonate (DMC) in a solvent in the presence of a base reagent and optionally an additive. The base reagent may be selected from MeMgCl, *i*PrMgCl, *i*PrMgBr, EtMgCl, nBuLi, and combinations thereof, such as *i*PrMgBr. The solvent may be selected from tetrahydrofuran (THF), toluene, 1,4-dioxane, 2-methyltetrahydrofuran (Me-THF), and combinations thereof, such as THF. The reaction temperature may be in the range from about 0 °C to about 60 °C. The temperature may be in the range from about 0 °C to about 30°C.

A compound of Formula V may be prepared according to a method represented by Scheme 9. The R groups are as defined anywhere in this disclosure.

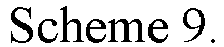

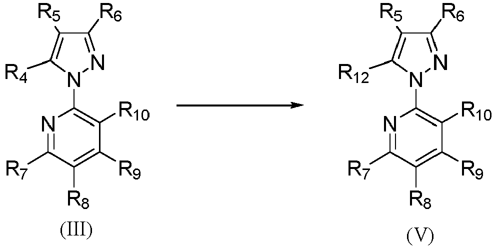

This method includes mixing a compound of Formula III with a cyanide reagent in an aprotic, polar solvent like N-methyl-2-pyrrolidone (NMP), acetonitrile, diglyme, triglyme, ethylene glycol, propylene glycol, ethanol, isobutanol, and alcohols and sulfolane, dimethyl carbonate, N,N-dimethyacetamide, and combinations thereof, such as diglyme. in the presence of copper salt and optionally an additive. The cyanide reagent may be selected from sodium cyanide, potassium cyanide, copper(I) cyanide, zinc cyanide, and combinations thereof, such as sodium cyanide. The copper salt may be selected from cuprous iodide, cuprous bromide, cuprous oxide, and combinations thereof, such as cuprous iodide. The additive may be potassium iodide. The reaction temperature may be in the range from about 120 °C to about 150 °C. The reaction temperature may be in the range from about 130 °C to about 140 °C.

A compound of Formula VI may be prepared according to a method represented by Scheme 10. The R groups are as defined anywhere in this disclosure.

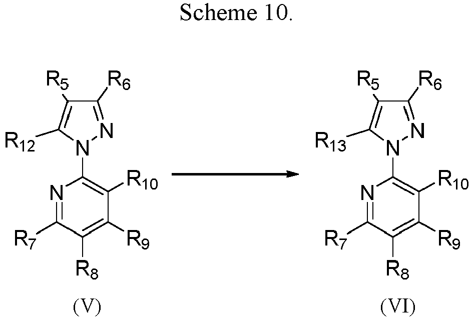

This method includes reacting a compound of Formula V with an aqueous metal hydroxide solution. The metal hydroxide may be selected from alkali hydroxide, alkaline earth metal hydroxide, and combinations thereof. The alkali hydroxide may be selected from lithium hydroxide, sodium hydroxide, potassium hydroxide, and combinations. The alkaline earth metal hydroxide may be selected from calcium hydroxide, barium hydroxide, and combinations thereof. The metal hydroxide may be sodium hydroxide or potassium hydroxide. The reaction temperature may be in the range from about 0 °C to about 90 °C. The reaction temperature may be in the range from about 60 °C to about 80 °C.

A compound of Formula VI may be prepared according to a method represented by Scheme 11. The R groups are as defined anywhere in this disclosure.

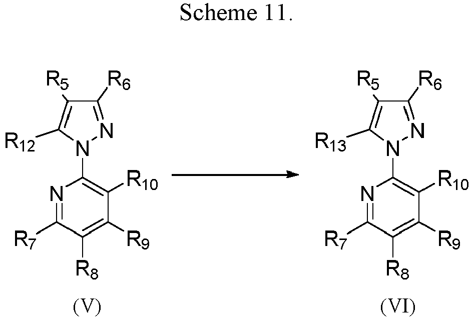

This method includes reacting 3-bromo-1-(3-chloropyridin-2-yl)-1H-pyrazole-5-carbonitrile in the presence of an aqueous acid solution. The acid may be selected from concentrated H₂SO₄, HCl, and combinations thereof, such as H₂SO₄. The reaction temperature may be in the range from about 50 °C to about 100 °C. The reaction temperature may be in the range from about 75 °C to about 85 °C.

### EXAMPLES

Without further elaboration, it is believed that one skilled in the art using the preceding description can utilize the present invention to its fullest extent. The following Examples are, therefore, to be construed as merely illustrative, and not limiting of the disclosure in any way whatsoever. The starting material for the following Examples may not have necessarily been prepared by a particular preparative run whose procedure is described in other Examples. It also is understood that any numerical range recited herein includes all values from the lower value to the upper value. For example, if a range is stated as 10-50, it is intended that values such as 12-30, 20-40, or 30-50, etc., are expressly enumerated in this specification. These are only examples of what is specifically intended, and all possible combinations of numerical values between and including the lowest value and the highest value enumerated are to be considered to be expressly stated in this application.

### Example 1. Hydrogen peroxide/HBr as a halogenation reagent.

34 grams of pyrazole and 505.8 g of 48% hydrogen bromide solution were charged to a reactor. 170 grams of 30% hydrogen peroxide was added drop-wise at 0 °C over 2 hours. The reaction temperature was controlled at 0-30 °C. After reaction, the product was precipitated as a solid, and then the reaction mixture was quenched with 10% sodium sulfite. After filtration and drying, 142 g of high purity (95%, LC Area) of 3,4,5-tribromo-1H-pyrazole was obtained.

### Example 2. Bromine/sodium hydroxide as a halogenation reagent.

34 grams of pyrazole was dissolved in water and then sodium hydroxide was added at 0 °C to obtain the corresponding pyrazole sodium salt. Next, 239.7 g of bromine was added drop-wise at 0 °C over 2 hours. The reaction temperature was controlled at 20-40 °C. After reaction, the product was precipitated as a solid, and then the reaction mixture was quenched with 10% sodium sulfite. After filtration and drying, 147 g of high purity (98%, LC Area) of 3,4,5-tribromo-1H-pyrazole was obtained.

### Example 3. Potassium iodide/sodium sulfite as a dehalogenation reagent.

100 grams of 3,4,5-tribromo-1H-pyrazole, 1.1 g of KI, and 62 g of Na₂SO₃ in 300 mL DMAc were reacted at 160-180 °C for 5 hours to completed reaction. After completion of the reaction, the reaction mixture was filtered, and then DMAc was distilled off under vacuum. Next, water was added to the crude product. The reaction mixture was stirred for 10 min. The product, 3,5-dibromo-1H-pyrazole, was precipitated as a solid. After filtration and drying, 68 g of high purity (98%, LC Area) of 3,5-dibromo-1H-pyrazole was obtained.

### Reference Example 4. Coupling reaction

22.6 grams of 3,5-dibromo-1H-pyrazole and 4.2 g of NaOH were reacted in the presence of toluene at a temperature of 150-160 °C. Water was removed by azetropic distillation under reflux temperature to yield the corresponding 3,5-dibromo-1H-pyrazole sodium salt. Then, DMAc and 15.5 g of 2,3-dichloropyridine were added and the mixture was reacted at 150-170 °C in an autoclave to produce 3-chloro-2-(3,5-dibromo-1H-pyrazol-1-yl)pyridine. After reaction, the reaction mixture was filtered. The DMAc solution containing 3-chloro-2-(3,5-dibromo-1H-pyrazol-1-yl)pyridine could be used in subsequent steps.

### Reference Example 5. Reaction in the presence of a transition metal catalyst.

0.95 grams of CuI, 3.32 g KI, and 5.4 g NaCN were added to a solution of 33.8 g 3-chloro-2-(3,5-dibromo-1H-pyrazol-1-yl)pyridine in DMAc at 10-25 °C. Next, the reaction mixture was stirred at 130-140 °C for 6 hours to complete reaction. DMAc was distilled off under vacuum. Toluene was added and stirred for 30 minutes. Next, the solution was filtered and toluene was removed under vacuum. After filtration and drying, 23.2 g (94%, LC Area) of 3-bromo-1-(3-chloropyridin-2-yl)-1H-pyrazole-5-carbonitrile was obtained.

### Reference Example 6. Acidification.

28.4 grams of high purity (94%, LC Area) 3-bromo-1-(3-chloropyridin-2-yl)-1H-pyrazole-5-carbonitrile was dissolved in 50% H₂SO₄ solution and charged to a flask. The mixture was heated to 80-85 °C and kept at this temperature for 3-4 hours to complete reaction. NaOH solution was used to adjust pH to a value in the range of about 9 to about 10 to precipitate the corresponding 5-bromo-2-(3-chloro-pyridin-2-yl)-2H-pyrazole-3-carboxylic acid sodium salt. H₂SO₄ was then used to adjust pH to a value in the range of about 1 to about 2 to precipitate 5-bromo-2-(3-chloro-pyridin-2-yl)-2H-pyrazole-3-carboxylic acid. After filtration and drying, 28.6 g (98%, LC Area) of 5-bromo-2-(3-chloro-pyridin-2-yl)-2H-pyrazole-3-carboxylic acid was obtained.

### Reference Example 7. Reaction in the presence of a Grignard reagent.

33.7 grams of 3-chloro-2-(3,5-dibromo-1H-pyrazol-1-yl)pyridine was dissolved in THF then iPrMgCl was added at 0 °C to yield the corresponding 3-chloro-2-(3,5-dibromo-1H-pyrazol-1-yl)pyridine magnesium salt. After 2.5 hours, 52.0 g of DMC was added drop-wise at room temperature over 6 hours. The reaction temperature was controlled at 20-40 °C. After reaction, THF and DMC were distilled off under reduced pressure, and then the reaction mixture was quenched with water. Next, toluene was added. After separation and concentration, 33 g of high purity (90%, LC Area) of methyl 3-bromo-1-(3-chloropyridin-2-yl)-1H-pyrazole-5-carboxylate was obtained.

### Reference Example 8. Hydrolysis.

33 grams of high purity (90%, LC Area) of methyl 3-bromo-1-(3-chloropyridin-2-yl)-1H-pyrazole-5-carboxylate, 12 g of 33% NaOH solution, and 90 g of toluene were charged to a flask and the mixture was heated to 80-85 °C and kept at this temperature for 1-2 hours to complete reaction. H₂SO₄ was used to adjust pH to a value in the range of about 1 to about 2 to precipitate 5-bromo-2-(3-chloro-pyridin-2-yl)-2H-pyrazole-3-carboxylic acid.

This written description uses examples to illustrate the present disclosure, including the best mode, and also to enable any person skilled in the art to practice the disclosure, including making and using any devices or systems and performing any incorporated methods. The scope of the disclosure is defined by the claims, and may include other examples that occur to those skilled in the art.

## Claims

1. A method of preparing a compound of Formula II, 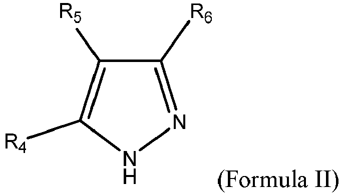
wherein each of R₄, R₅, and R₆ is independently selected from hydrogen and halogen; and
wherein at least one of R₄, R₅, and R₆ is hydrogen, the method comprising
I) forming a mixture comprising
A) a compound of Formula I, 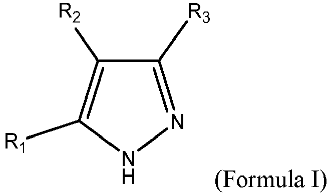
wherein each of R₁, R₂, and R₃ is independently a halogen; and
wherein the compound of Formula I is prepared according to a method comprising
i) forming a mixture comprising
a) pyrazole or a pyrazole derivative;
b) a halogenation reagent;
c) water;
d) optionally a solvent; and
e) optionally an inorganic base; and
ii) reacting the mixture;
B) optionally a dehalogenation reagent;
C) a reducing agent; and
D) a solvent; and
II) reacting the mixture.

2. A method of preparing a compound of Formula II-A, 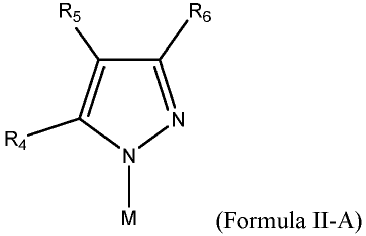
wherein M is selected from alkali metals and alkaline metals;
each of R₄, R₅, and R₆ is independently selected from hydrogen and halogen; and
wherein at least one of R₄, R₅, and R₆ is hydrogen, the method comprising
I) forming a mixture comprising
A) a compound of Formula I, 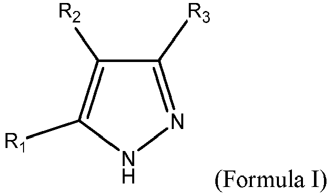
wherein each of R₁, R₂, and R₃ is independently a halogen; and
wherein the compound of Formula I is prepared according to a method comprising
i) forming a mixture comprising
a) pyrazole or a pyrazole derivative;
b) a halogenation reagent;
c) water;
d) optionally a solvent; and
e) optionally an inorganic base; and
ii) reacting the mixture;
B) optionally a dehalogenation reagent;
C) a reducing agent; and
D) a solvent; and
II) reacting the mixture.

## Patentansprüche

1. Verfahren zur Herstellung einer Verbindung der Formel II, 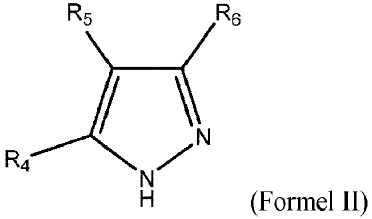
wobei R₄, R₅ und R₆ jeweils unabhängig aus Wasserstoff und Halogen ausgewählt sind; und
wobei mindestens einer der Reste R₄, R₅ und R₆ für Wasserstoff steht, wobei das Verfahren Folgendes umfasst:
I) das Bilden einer Mischung, umfassend
A) eine Verbindung der Formel I, 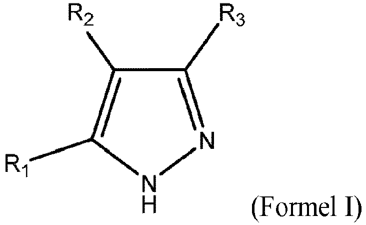
wobei R₁, R₂ und R₃ jeweils unabhängig für ein Halogen stehen; und
wobei die Verbindung der Formel I nach einem Verfahren hergestellt wird, das Folgendes umfasst:
i) das Bilden einer Mischung, umfassend
a) Pyrazol oder ein Pyrazolderivat
b) ein Halogenierungsreagenz,
c) Wasser;
d) gegebenenfalls ein Lösungsmittel; und
e) gegebenenfalls eine anorganische Base; und
ii) das Umsetzen der Mischung;
B) gegebenenfalls ein Dehalogenierungsreagenz;
C) ein Reduktionsmittel; und
D) ein Lösungsmittel; und
II) das Umsetzen der Mischung.

2. Verfahren zur Herstellung einer Verbindung der Formel II-A, 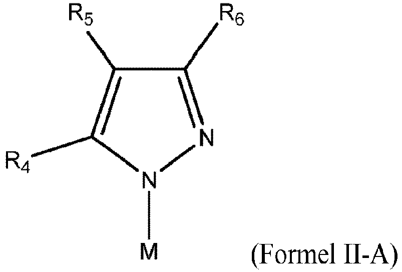
wobei M aus Alkalimetallen und alkalischen Metallen ausgewählt ist;
R₄, R₅ und R₆ jeweils unabhängig aus Wasserstoff und Halogen ausgewählt sind; und
wobei mindestens einer der Reste R₄, R₅ und R₆ für Wasserstoff steht, wobei das Verfahren Folgendes umfasst:
I) das Bilden einer Mischung, umfassend
A) eine Verbindung der Formel I, 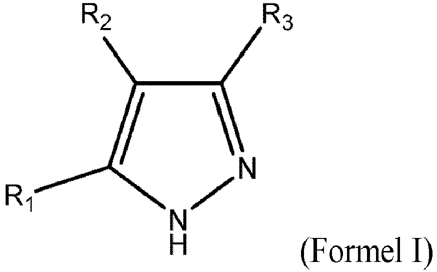
wobei R₁, R₂ und R₃ jeweils unabhängig für ein Halogen stehen; und
wobei die Verbindung der Formel I nach einem Verfahren hergestellt wird, das Folgendes umfasst:
i) das Bilden einer Mischung, umfassend
a) Pyrazol oder ein Pyrazolderivat;
b) ein Halogenierungsreagenz;
c) Wasser;
d) gegebenenfalls ein Lösungsmittel; und
e) gegebenenfalls eine anorganische Base; und
ii) das Umsetzen der Mischung;
B) gegebenenfalls ein Dehalogenierungsreagenz;
C) ein Reduktionsmittel; und
D) ein Lösungsmittel; und
II) das Umsetzen der Mischung.

## Revendications

1. Procédé de préparation d'un composé de formule II, 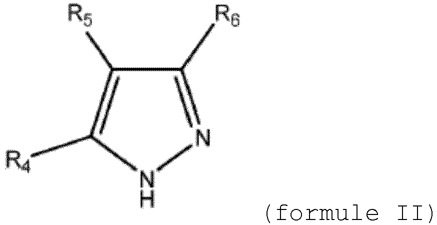
chacun parmi R₄, R₅, et R₆ étant indépendamment choisi parmi hydrogène et halogène ; et
au moins l'un parmi R₄, R₅, et R₆ étant hydrogène, le procédé comprenant
I) la formation d'un mélange comprenant
A) un composé de formule I, 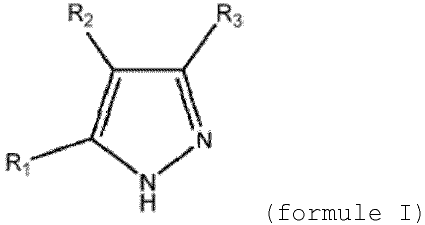
chacun parmi R₁, R₂ et R₃ étant indépendamment un halogène ; et
le composé de formule I étant préparé selon un procédé comprenant
i) la formation d'un mélange comprenant
a) le pyrazole ou un dérivé de pyrazole ;
b) un réactif d'halogénation ;
c) de l'eau ;
d) éventuellement un solvant ; et
e) éventuellement une base inorganique ; et
ii) la mise en réaction du mélange ;
B) éventuellement un réactif de déshalogénation ;
C) un agent de réduction ; et
D) un solvant ; et
II) la mise en réaction du mélange.

2. Procédé de préparation d'un composé de formule II-A, 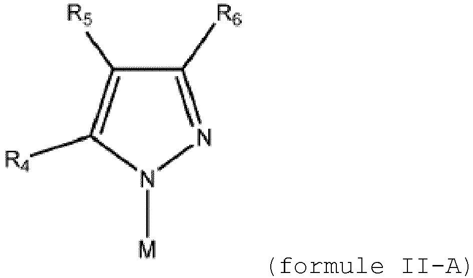
M étant choisi parmi des métaux alcalins et des métaux alcalino-terreux ;
chacun parmi R₄, R₅, et R₆ étant indépendamment choisi parmi hydrogène et halogène ; et
au moins l'un parmi R₄, R₅, et R₆ étant hydrogène, le procédé comprenant
I) la formation d'un mélange comprenant
A) un composé de formule I, 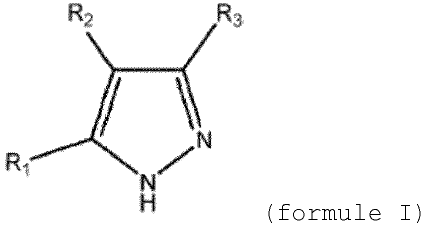
chacun parmi R₁, R₂ et R₃ étant indépendamment un halogène ; et
le composé de formule I étant préparé selon un procédé comprenant
i) la formation d'un mélange comprenant
a) le pyrazole ou un dérivé de pyrazole ;
b) un réactif d'halogénation ;
c) de l'eau ;
d) éventuellement un solvant ; et
e) éventuellement une base inorganique ; et
ii) la mise en réaction du mélange ;
B) éventuellement un réactif de déshalogénation ;
C) un agent de réduction ; et
D) un solvant ; et
II) la mise en réaction du mélange.
